# EUROPÄISCHE PATENTSCHRIFT

(11) **EP 0 507 093 B1**
(45) Veröffentlichungstag und Bekanntmachung des Hinweises auf die Patenterteilung: **19.06.1996**
(21) Anmeldenummer: 92103663.8
(22) Anmeldetag: 04.03.1992
(51) Int. Cl.: C07D 239/42, A01N 47/36, C07D 239/47, C07D 239/52, C07D 251/16, C07D 251/46, C07D 251/22, C07C 311/55, C07C 307/02, C07C 311/48

(54) **Verfahren zur Herstellung von Sulfonylharnstoffen**
Process for the preparation of sulfonylureas
Procédé pour la préparation de sulfonylurées

(30) Priorität: 07.03.1991 DE 4107326
(43) Veröffentlichungstag der Anmeldung: 07.10.1992
(73) Patentinhaber: Hoechst Schering AgrEvo GmbH, D-13342 Berlin (DE)
(72) Erfinder: Willms, Lothar, Dr., W-5416 Hillscheid (DE); Lachhein, Stephen, Dr., W-6238 Hofheim am Taunus (DE); Schlegel, Günter, Dr., W-6237 Liederbach (DE); Kehne, Heinz, Dr., W-6238 Hofheim am Taunus (DE)

(56) Entgegenhaltungen:
- EP-A- 0 131 258
- EP-A- 0 158 248
- EP-A- 0 342 569
- EP-A- 0 353 641
- EP-A- 0 409 114
- PHOSPHORUS, SULFUR, AND SILICON RELAT. ELEM., Band 61, Nr. 1-2, 6. Juni 1991, Seiten 19-25; M.I.R. HEDAYATULLAH et al.: "Synthèses a l'aide d'hétérocumulènes. 8. Elaboration d'une nouvelle classe de fongicides renfermant les enchaînements mixtes sulfamate et carbamate"
- CHEMISCHE BERICHTE, Band 96, 1963, Seiten 56-67, Weinheim, DE; R. GRAF: "Alkohole und Phenole"

## Beschreibung

Die Erfindung betrifft Verfahren zur Herstellung von Herbiziden aus der Gruppe der heterocyclisch substituierten Sulfonylharnstoffe, speziell der Verbindungen der Formel I, 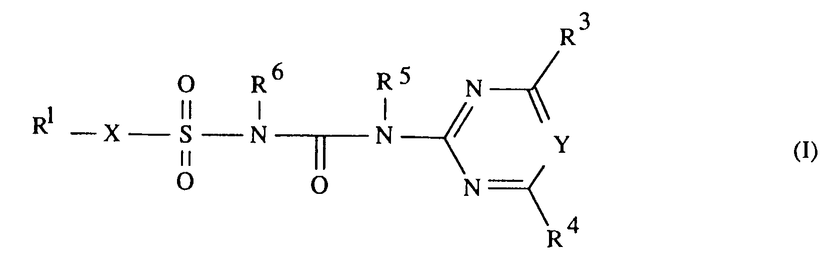 worin
- X: Sauerstoff, -O-NR²- oder -SO₂-NR²-, wobei O bzw. SO₂ aus den beiden letztgenannten divalenten Gruppen direkt an R¹ gebunden sind,
- Y: Stickstoff oder CH,
- R¹: (C₁-C₆)-Alkyl, (C₂-C₆)-Alkenyl oder (C₂-C₆)-Alkinyl, wobei jeder der genannten 3 Reste unsubstituiert oder ein- oder mehrfach durch Reste aus der Gruppe Halogen, (C₁-C₄)-Alkoxy und (C₁-C₄)-Alkoxycarbonyl substituiert ist,
oder, im Falle von X = Sauerstoff, auch Phenyl, das unsubstituiert oder durch ein oder mehrere Reste aus der Gruppe Halogen, Nitro, (C₁-C₄)-Alkyl, (C₁-C₄)-Halogenalkyl, (C₁-C₄)-Alkoxy, (C₁-C₄)-Halogenalkoxy oder (C₁-C₄)-Alkoxycarbonyl substituiert ist,
- R²: Wasserstoff, (C₁-C₆)-Alkyl, (C₂-C₆)-Alkenyl, (C₂-C₆)-Alkenyl oder (C₃-C₆)-Cycloalkyl,
- R³,R⁴: unabhängig voneinander Wasserstoff, (C₁-C₄)-Alkyl oder (C₁-C₄)-Alkoxy, wobei jeder der letztgenannten zwei Reste unsubstituiert oder ein- oder mehrfach durch Reste aus der Gruppe Halogen, Alkoxy und Alkylthio substituiert ist, oder Halogen, (C₁-C₄)-Alkylthio, (C₁-C₄)-Alkylamino oder Di-[(C₁-C₄)-alkyl]-amino und
- R⁵,R⁶: unabhängig voneinander Wasserstoff oder (C₁-C₄)-Alkyl
bedeuten, sowie deren physiologisch verträglichen Salze mit Säuren oder, falls mindestens einer der Reste R⁵ und R⁶ Wasserstoff ist, mit Basen.

Verbindungen der Formel I sind bekannt und werden als Pflanzenschutzmittel mit herbizider Wirkung eingesetzt; siehe EP-A-0131258 (US-A-4,601,747), EP-A-0342569 (ZA-A-89/3643) und EP-A-4163 (US-A-4,191,553). Darin sind auch einige Verfahren zitiert oder beschrieben, nach denen Verbindungen der Formel I hergestellt werden können.

Nachteilig bei den bekannten Verfahren sind die relativ niedrigen Ausbeuten von höchstens etwa 65-70%. Dadurch bedingt fallen vergleichsweise große Mengen an Verunreinigungen und Nebenprodukten an, die bei Anwendung im großtechnischen Maßstab Abfall bedeuten, der aufwendig entsorgt werden muß, z. B. durch Verbrennung. Die bekannten Verfahren sind deshalb aus ökologischer als auch ökonomischer Sicht für die Durchführung im industriellen, großtechnischen Maßstab ungünstig. Außerdem tritt bei solch niedriger Ausbeute ein drastischer Verlust an eingesetzten Ausgangsmaterialien ein, was die Wirtschaftlichkeit der Verfahren schmälert.

Es wurde nun ein neues Verfahren gefunden, nach dem die Verbindungen der Formel I in überraschend hoher Ausbeute und Reinheit herstellbar sind und das sich für die Durchführung im großtechnischen Maßstab eignet.

Gegenstand der vorliegenden Erfindung ist ein Verfahren zur Herstellung der genannten Verbindungen der Formel I oder deren Salze, dadurch gekennzeichnet, daß man Verbindungen der Formel II, 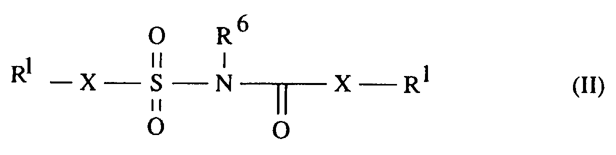 worin R¹, R⁶ und X wie in Formel I definiert sind, mit Verbindungen der Formel III, 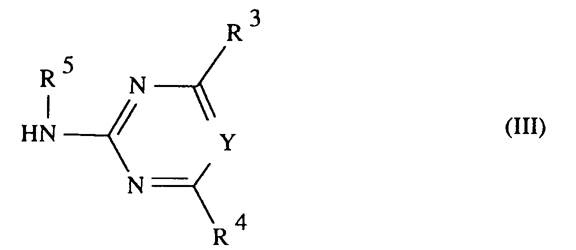 worin R³, R⁴, R⁵ und Y wie in Formel I definiert sind, umsetzt.

In den genannten Formeln bedeutet Alkyl geradkettiges oder verzweigtes Alkyl; dies gilt entsprechend für das Kohlenwasserstoffteil in den übrigen Resten, wie Alkoxy, Haloalkyl, Haloalkoxy, Alkylcarbonyl, Alkylamino, Alkenyl, Alkinyl, Alkylsulfonyl usw. Halogen bedeutet Fluor, Chlor, Brom oder Jod, vorzugsweise Fluor, Chlor oder Brom. Haloalkyl bedeutet Alkyl, das durch ein oder mehrere Halogenatome substituiert ist; entsprechendes gilt für Haloalkoxy.

Unter den erfindungsgemäßigen Verfahren zur Herstellung der Verbindungen der Formel I sind diejenigen von besonderem Interesse, in denen R¹X N-(C₁-C₆)-Alkylsulfonyl-N-(C₁-C₃)-alkylamino oder (C₁-C₄)-Alkoxyphenoxy, R³ und R⁴ unabhängig voneinander (C₁-C₂-Alkyl oder (C₁-C₂)-Alkoxy, R⁵ Wasserstoff und R⁶ Wasserstoff oder Methyl bedeuten. Vorzugsweise ist dabei R¹X N-[(C₁-C₃)-Alkylsulfonyl]-N-[(C₁-C₂)-alkyl]-amino, insbesondere N-(Methylsulfonyl)-N-(methyl)-amino, N-(Methylsulfonyl)-N-(ethyl)-amino, N-(Ethylsulfonyl)-N-(methyl)-amino oder N-(Ethylsulfonyl)-N-(ethyl)-amino, oder (C₁-C₃)-Alkoxyphenoxy, insbesondere 2-Methoxy-phenoxy, 2-Ethoxy-phenoxy, 2-n-Propoxy-phenoxy oder 2-Isopropoxy-phenoxy, und R³ und R⁴ unabhängig voneinander (C₁-C₂)-Alkyl oder (C₁-C₂)-Alkoxy, insbesondere Methyl oder Methoxy, und R⁵ und R⁶ Wasserstoff oder Methyl.

Die Ausbeuten nach dem erfindungsgemäßen Verfahren liegen vergleichsweise hoch, z. B. bei 95 % d.Th. und mehr, wobei die Reinheiten der entstehenden Sulfonylharnstoffe der Formel I oft größer als 95 Gew.-% sind.

Das erfindungsgemäße Verfahren wird in der Regel in Gegenwart von unter den Reaktionsbedingungen inerten anorganischen oder organischen Lösungsmittel oder Gemischen davon durchgeführt. Geeignet als Lösungsmittel sind inerte organische Lösungsmittel, aber auch Wasser und wäßrige organische Lösungsmittel.

Beispiele für geeignete Lösungsmittel sind aliphatische oder aromatische, gegebenenfalls halogenierte Kohlenwasserstoffe, aprotische polare organische Lösungsmittel, wie Dialkylalkanoylamide, Dialkylsulfoxide, Polyalkylenglykoldialkylether, N-alkylierte cyclische Amide und Nitrile sowie Mischungen aus genannten Lösungsmitteln.

Bevorzugt sind Lösungsmittel wie z. B. Toluol, Xylol, Chlorbenzol, 1,2-Dichlorethan, Dimethylformamid, Dimethylsulfoxid, Di-, Tri- oder Tetraethylenglykoldialkylether, insbesondere -dimethyl oder -diethylether, N-Methylpyrrolidon, Acetonitril oder auch Mischungen aus zwei oder mehreren der genannten Lösungsmittel.

Das erfindungsgemäße Verfahren kann aber auch in wäßrigem, z. B. rein wäßrigem Medium durchgeführt werden.

Es ist in der Regel vorteilhaft, die Verbindung der Formel II im Verhältnis zur Verbindung der Formel III äquimolar oder in geringem Überschuß einzusetzen. Bevorzugt ist ein Molverhältnis für II:III von 1:1 bis 1,2:1, insbesondere 1:1 bis 1,1:1.

Die Reaktionstemperaturen liegen vorzugsweise bei 0°C bis zum Siedepunkt des eingesetzten Lösungsmittels, insbesondere bei Raumtemperatur (z. B. 20°C) bis 110°C.

Ein Vorteil des erfindungsgemäßen Verfahrens besteht darin, daß die aus den Verbindungen der Formel II abgespaltene Verbindung der Formel IV, 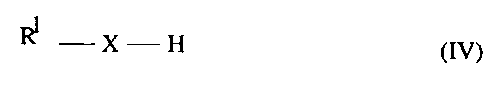 worin R¹ und X wie oben definiert sind, bei der Durchführung des erfindungsgemäßen Verfahrens wieder quantitativ recyclisiert werden kann und bei nachfolgenden Synthesen zu den Verbindungen der Formel II wieder direkt eingesetzt werden kann. Gegcbenenfalls können die Verbindungen der Formel IV vor dem Recycling gereinigt werden, z. B. in einfacher Weise durch Destillation.

Ein weiterer Vorteil dieses Verfahrens besteht in der Vermeidung des Einsatzes der Verbindungen der Formel V, 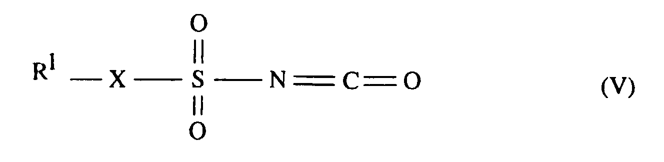 die bei herkömmlichen Verfahren zur Herstellung von Verbindungen der Formel I verwendet werden und dabei unter üblichen Reaktionstemperaturen von mehr als 110 °C intermediär gebildet werden. Unter diesen üblichen Bedingungen können die Isocyanate der Formel V jedoch thermisch sehr labil sein und teilweise zerfallen, was sich dann in niedrigen Ausbeuten widerspiegelt (s. EP-A-0131258 (US-A-4,601,747), DE-A-2257240 (US-A-3,931,277), G. Lohaus, Chem. Ber. 105, 2791-2799 (1972)).

Ein zusätzlicher Vorteil des erfindungsgemäßen Verfahrens besteht darin, daß die Lösungsmittel in nahezu quantitativer Ausbeute wieder recyclisiert werden können, da die Produkte der Formel I als schwerlösliche Verbindungen aus den Reaktionsmedium in hoher Reinheit und Ausbeute ausfallen. Die Lösungsmittel können anschließend z. B. durch Destillation gereinigt und dann wieder in den Prozeß eingeführt werden.

Die für die Herstellung der Verbindungen der allgemeinen Formel I nach dem erfindungsgemäßen Verfahren benötigten Ausgangsverbindungen der Formeln II und III lassen sich nach zum Teil literaturbekannten Verfahren herstellen.

So sind die Heterocyclen der Formel III entweder käuflich oder lassen sich nach geeigneten Labormethoden leicht herstellen; siehe z. B. US-A-4,310,470; EP-A-0027200; US-A-4,299,960; M. J. Langermann, C.K. Banks, J. Am. Chem. Soc. 73, 3011 (1951).

Die Verbindungen der Formel II sind teilweise neu und können analog üblichen Methoden (siehe z. B. Tietze und Eicher in "Reaktionen und Synthesen", S. 92, Thieme Verlag, Stuttgart 1981) durch Umsetzung der entsprechenden Sulfonamide VI mit den entsprechenden Säurechloriden VII erhalten werden, 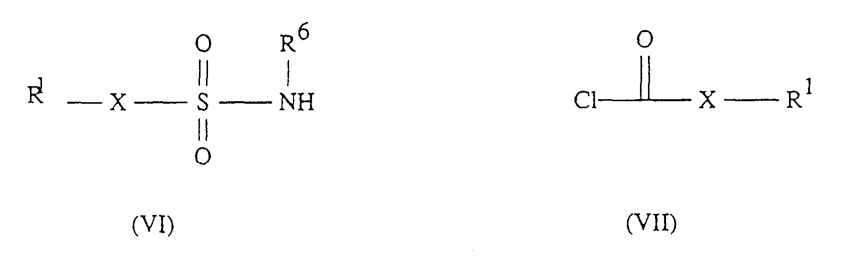 die ihrerseits wiederum durch Umsetzung der Verbindung der Formel R¹-X-H (genannte Formel IV) und den entsprechenden Amidosulfochloriden der Formel Cl-SO₂-NHR⁶ (ergibt Produkt der Formel VI) bzw. Phosgen oder Chlorameisensäureester (ergibt Produkt der Formel VII) nach laborüblichen Methoden zugänglich sind (siehe z. B. "Organikum", 7. Auflage, S. 539, VEB Deutscher Verlag der Wissenschaften, Berlin 1967).

Aus Chem. Ber. 96 (1963) 56-57 ist bereits bekannt, Alkohole wie p-Chlorphenol mit der halbmolaren Menge an Chlorsulfonylisocyanat zur Verbindung der Formel (II), worin X = O, R¹ = p-Chlorphenyl, R⁶ = H bedeuten, umzusetzten. Für Verbindungen der Formel X = -ONR²- oder -SO₂-NR² ist das Verfahren neu und ebenfalls Gegenstand der Erfindung.

Die Verbindungen der Formel II können nach diesem Verfahren in hervorragender Ausbeute hergestellt werden. Das Verfahren ist dadurch gekennzeichnet, daß man Verbindungen der Formel IV mit der etwa halbmolaren Menge an Chlorsulfonylisocyanat umsetzt:

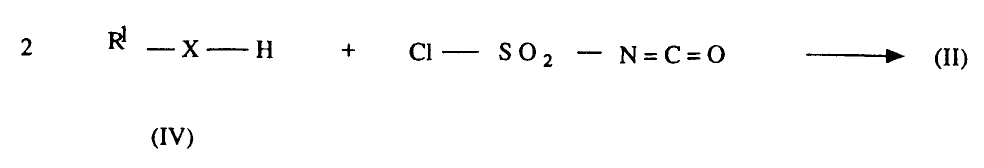

Dabei können die Verbindungen der Formel IV entweder als freie Alkohole, Hydroxylamine bzw. Amide (je nach Bedeutung von R¹ und X), gegebenenfalls in Gegenwart einer organischen oder anorganischen Base, oder als eine Mischung von freier Verbindung IV und einem entsprechenden Salz der Verbindung IV eingesetzt werden. Im letztgenannten Fall ist eine Mischung von freier Verbindung IV mit einem Alkalimetallsalz der Verbindung IV, wie das Natrium- oder Kaliumsalz, im Molverhältnis von etwa 1:1 bevorzugt.

In der Regel wird die Verbindung der Formel IV zu dem Chlorsulfonylisocyanat im molaren Verhältnis von 1,0:0,5 bis 2,0:0,5, vorzugsweise 1,0:0,5 bis 1,5:0,5 eingesetzt.

Es ist meistens angebracht, die Umsetzung in Gegenwart eines inerten Lösungsmittels durchzuführen, wobei die bei der obengenannten Umsetzung der Verbindungen der Formeln II und III genannten inerten organischen Lösungsmittel und Lösungmitteltypen auch hier geeignet sind.

Das neue Verfahren für die Darstellung der Verbindungen II kann derart durchgeführt werden, daß man die Verbindungen der Formel IV mit 0,5 Moläquivalenten einer anorganischen Base, z. B. eines Alkalihydroxids oder Alkalialkoholats, in eine 1:1 Mischung, bezogen auf molare Mengen, aus freier Verbindung IV und deren Alkalisalz überführt und anschließend mit 0,5 Moläquivalenten von Chlorosulfonylisocyanat reagieren läßt. Alternativ kann man die freie Verbindung IV mit 0,5 Moläquivalenten Chlorsulfonylisocyanat reagieren lassen und anschließend eine Base, beispielsweise eine organische Base (z. B. ein tertiäres Amin wie Triethylamin) zusetzen und die Umsetzung bis zum Verbrauch der Verbindung IV weiterführen. Auch ist es in manchen Fällen möglich, die vollständige Umsetzung ohne Base durch Reaktion der freien Verbindung IV mit 0,5 Moläquivalenten Chlorsulfonylisocyanat unter Erhitzen der Reaktionsmischung bis zum Siedepunkt des Lösungsmittels und gegebenenfalls Abdestillieren der entstehenden HCl zu bewirken.

Die Reaktionstemperaturen für die Darstellung der Verbindungen der Formel II hängen von den speziellen Verbindungen der Formel IV und der Verfahrensvariante ab und sind meist bei 0°C bis 140°C, vorzugsweise bei 20°C bis 130°C.

Bevorzugt ist die Umsetzung einer 1:1-Mischung von freier Verbindung der Formel IV mit einem Alkalimetallsalz der Verbindung der Formel IV. Die letztere Verfahrensvariante ergibt hohe Ausbeuten und gelingt überraschenderweise schon bei für Chlorsulfonylisocyanat günstig niedrige Temperaturen von weniger als 100 °C, vorzugsweise 50 bis 90 °C.

In einer besonders vorteilhaften Ausgestaltung der Erfindung kann die gesamte Reaktionssequenz zur Herstellung einer Verbindung der Formel I ohne Isolierung des Zwischenprodukts der Formel II direkt aus den Ausgangsverbindungen der Formel IV und Chlorsulfonylisocyanat, vorzugsweise in einer Eintopfreaktion, durchgeführt werden.

Gegenstand der Erfindung ist deshalb auch die Kombination der genannten Teilverfahren, dadurch gekennzeichnet, daß man
a) die Umsetzung der Verbindung der Formel IV und Chlorsulfonylisocyanat im genannten Molverhältnis zur Verbindung der Formel II durchführt und, vorzugsweise ohne Isolierung des Zwischenprodukts der Formel II,
b) die Umsetzung der Verbindungen der Formeln II und III zur Verbindung der Formel I anschließt.

Als überraschend ist der glatte Verlauf des erfindungsgemäßen Verfahrens und die hohe Ausbeute anzusehen, weil das Ausgangsprodukt der Formel II mehrere aktivierte, elektrophile und nucleophile Zentren enthält. Insbesondere alle der elektrophilen Zentren können prinzipiell mit den nucleophilen Substanzen der Formel III reagieren und so durch Fragmentierungsreaktionen eine Vielzahl von Nebenprodukten liefern; vgl. Beyer, Lehrbuch der org. Chemie, 19. Auflage, S. 128, Hirzel Verlag Stuttgart, wonach Sulfonylgruppen und Phenoxygruppen sehr gute Abgangsgruppen darstellen.

Als besonders überraschend ist das erfindungsgemäße Verfahren mit Verbindungen der Formel II, worin X = -SO₂NR²- oder -ONR²- bedeutet, da hierbei in Verfahrenstufe b) eine Umamidierung stattfindet, die nahezu quantitativ zum gewünschten Produdukt führt; dagegen wäre eher eine Mischung aus den Verbindungen der Formeln II und I im Verhältnis von etwa 1:1 zu erwarten gewesen. Die Nebenreaktionen und die erwartete Mischung treten aber erstaunlicherweise nach dem erfindungsgemäßen Verfahren praktisch nicht auf, sondern es werden meist Ausbeuten von über 95 % d. Th. und Reinheiten von über 95 % erhalten. Somit stellt das erfindungsmäße Verfahren einen neuen, einfachen, auch im größeren technischen Maßstab gut reproduzierbaren und hochselektiven Prozeß zur Darstellung der Verbindungen der Formel I in nahezu quantitativen Ausbeuten dar. Das Verfahren kann kontinuierlich oder diskontinuierlich durchgeführt werden.

### BEISPIEL 1 1-[(N-Methylsulfonyl-N-methyl-amino)-sulfonyl]-3-(4,6-dimethoxy-2-pyrimidyl)-harnstoff

32,3 g 1-[(N-Methylsulfonyl-N-methylamino)-sulfonyl]-3-methyl-sulfonyl-3-methylharnstoff werden in 500 ml Chlorbenzol aufgeschlämmt und bei 80°C unter Rühren mit 15,5 g 2-Amino-4,6-dimethoxy-pyrimidin versetzt und für 3 Stunden auf 80°C erhitzt. Nach Abkühlen auf 20°C wird der Niederschlag abfiltriert und mit 100 ml Chlorbenzol gewaschen. Man erhält 36,4 g des gewünschten Produkts mit einer Reinheit von 98,1 %, was einer Ausbeute von 96,8 % d. Th. entspricht. Der Schmelzpunkt für das Produkt liegt bei 183-185°C. 5,1 g Methansulfonsäure-N-methylamid werden durch Destillation aus dem Filtrat zurückgewonnen, was einer Ausbeute von 92,7 % d. Th. entspricht.

### BEISPIEL 2 1-(2-Ethoxyphenoxysulfonyl)-3-(4,6-dimethoxy-2-pyrimidyl)-harnstoff

38,1 g (2-Ethoxyphenoxysulfonyl)-(2-ethoxyphenoxy)-carbamat werden in 500 ml Toluol gelöst und bei Raumtemperatur mit 15,5 g 2-Amino-4,6-dimethoxypyrimidin versetzt und für 2 Stunden bei 100°C erhitzt. Nach Abkühlen auf 30°C wird der Niederschlag abfiltriert und mit 100 ml Toluol gewaschen. Es werden 38,8 g des gewünschten Produkts mit einer Reinheit von 98,8 % erhalten, was einer Ausbeute von 96,4 % d. Th. entspricht. Der Schmelzpunkt für das Produkt liegt bei 147-149°C. 6,5 g 2-Ethoxyphenol werden durch Destillation aus der Mutterlauge zurückgewonnen.

Analog den Beispielen 1 und 2 können die in nachfolgender Tabelle 1 aufgeführten Verbindungen der Formel Ia synthetisiert werden.

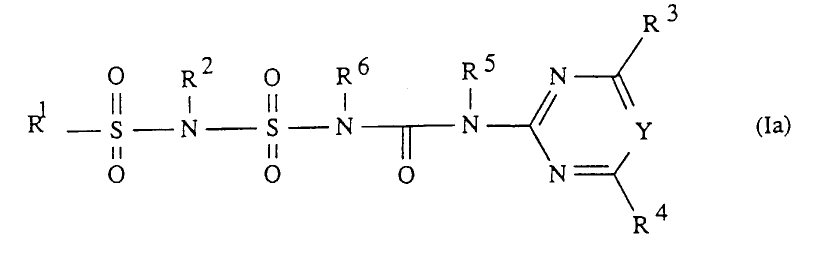

**Tabelle 1:**

| Verbindungen der Formel (Ia) | | | | | | | | |
|---|---|---|---|---|---|---|---|---|
| Bsp. | R¹ | R² | R³ | R⁴ | R⁵ | R⁶ | Y | Smp. [°C] |
| 3 | CH₃ | C₃H₇ | CH₃ | CH₃ | H | H | CH | 156-157 |
| 4 | CH₃ | CH(CH₃)₂ | CH₃ | CH₃ | H | H | CH | 122-123 |
| 5 | C₂H₅ | C₂H₅ | CH₃ | CH₃ | H | H | CH | |
| 6 | CH₃ | CH₃ | CH₃ | CH₃ | H | H | CH | |
| 7 | CH₃ | CH₃ | CH₃ | CH₃ | H | CH₃ | CH | |
| 8 | C₄H₉ | CH₃ | CH₃ | CH₃ | H | H | CH | |
| 9 | CH₃ | c-C₆H₁₁ | CH₃ | CH₃ | H | H | CH | |
| 10 | C₂H₅ | C₂H₅ | OCH₃ | OCH₃ | H | H | CH | 148 |
| 11 | C₂H₅ | CH₃ | OCH₃ | OCH₃ | H | H | CH | |
| 12 | C₂H₅ | CH₃ | Cl | CH₃ | H | H | CH | 110-112 |
| 13 | CH₂Cl | CH₃ | OCH₃ | CH₃ | H | H | CH | |
| 14 | CH₃ | CH₃ | CH₃ | OCH₃ | CH₃ | H | N | |
| 15 | CH₃ | C₃H₇ | CH₃ | OCH₃ | H | H | N | |
| 16 | CH₃ | CH(CH₃)₂ | CH₃ | CH₃ | H | H | N | |
| 17 | C₂H₅ | C₂H₅ | CH₃ | CH₃ | H | H | N | |
| 18 | C₂H₅ | CH₃ | OCH₃ | OCH₃ | H | H | N | |
| 19 | C₂H₅ | C₂H₅ | OCH₃ | OCH₃ | H | H | N | |
| 20 | C₉H₄ | CH₃ | CH₃ | CH₃ | H | H | N | |

### BEISPIEL 21 (Herstellung von Verbindungen der Formel II)

### 1-[(N-Methylsulfonyl-N-methylamino)-sulfonyl]-3-methylsulfonyl-3-methyl-harnstoff

109 g Methansulfonsäure-N-methylamid werden in 1000 ml Chlorbenzol gelöst und mit 20 g Natriumhydroxid versetzt und das entstehende Reaktionswasser (9 ml) ausgekreist. Anschließend werden bei 80°C 70,0 g Chlorsulfonylisocyanat tropfenweise zu der Reaktionslösung gegeben und für 3 Stunden bei 90°C nacherhitzt. Während der Reaktionszeit fällt das entstehende Natriumchlorid an. Die Reaktionsmischung wird bei Raumtemperatur filtriert und der Filterrückstand vom Natriumchlorid frei gewaschen.

Man erhält 157 g Produkt mit einer Reinheit von 97,2 %, was einer Ausbeute von 94,5 % d. Th. entspricht. Der Schmelzpunkt beträgt 128-129°C und ist mit einer auf einem alternativen Syntheseweg hergestellten Produkt identisch.

### BEISPIEL 22 (Eintopfverfahren)

### 1-(2-Ethoxyphenoxysulfonyl)-3-(4,6-dimethoxy-2-pyrimidyl)-harnstoff

138 g 2-Ethoxyphenol werden in 1000 ml Chlorbenzol gelöst und mit 20 g Natriumhydroxid versetzt und das entstehende Reaktionswasser (9 ml) abdestilliert. Danach werden bei 50°C 70,5 g Chlorsulfonylisocyanat tropfenweise zugegeben und für 3 Stunden bei 100°C erhitzt. Während der Reaktionszeit fällt das entstehende Natriumchlorid an. Die Reaktionstemperatur wird auf 80°C abgesenkt; danach versetzt man mit 77,5 g 2-Amino-4,6-dimethoxypyrirnidin, Nach 4 Stunden Nachrühren bei 80°C wird auf Raumtemperatur abgekühlt und filtriert Das noch vorhandene Kochsalz wird mit Wasser aus dem Filtrat herausgewaschen. Es verbleiben 186,5 g des oben bezeichneten Sulfonylharnstoffs mit einer Reinheit von 97,8 %, was einer Ausbeute von 91,6 % d. Th. entspricht. Der Schmelzpunkt des Produkts liegt bei 146-148°C. 63,2 g 2-Ethoxyphenol werden durch Destillation aus der Mutterlauge zurückgewonnen.

## Patentansprüche

1. Verfahren zur Herstellung von Verbindungen der Formel (I) oder deren Salzen, 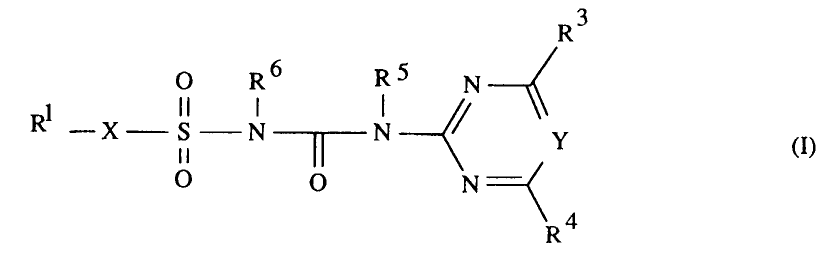 worin
X Sauerstoff, -O-NR²- oder -SO₂-NR²- wobei O bzw. SO₂ aus den beiden letztgenannten divalenten Gruppen direkt an R¹ gebunden sind,
Y Stickstoff oder CH ,
R¹ (C₁-C₆)-Alkyl, (C₂-C₆)-Alkenyl oder (C₂-C₆)-Alkinyl, wobei jeder der genannten 3 Reste unsubstituiert oder ein- oder mehrfach durch Reste aus der Gruppe Halogen, (C₁-C₄)-Alkoxy und (C₁-C₄)-Alkoxycarbonyl substituiert ist,
oder, im Falle von X = Sauerstoff, auch Phenyl, das unsubstituiert oder durch ein oder mehrere Reste aus der Gruppe Halogen, Nitro, (C₁-C₄)-Alkyl, (C₁-C₄)-Halogenalkyl, (C₁-C₄)-Alkoxy, (C₁-C₄)-Halogenalkoxy oder (C₁-C₄)-Alkoxycarbonyl substituiert ist,
R² Wasserstoff, (C₁-C₆)-Alkyl, (C₂-C₆)-Alkenyl, (C₂-C₆)-Alinyl oder (C₃-C₆)-Cycloalkyl,
R³, R⁴ unabhängig voneinander Wasserstoff, (C₁-C₄)-Alkyl oder (C₁-C₄)-Alkoxy, wobei jeder der letztgenannten zwei Reste unsubstituiert oder ein- oder mehrfach durch Reste aus der Gruppe Halogen, Alkoxy und Alkylthio substituiert ist, oder Halogen, (C₁-C₄)-Alkylthio, (C₁-C₄)-Alkylamino oder Di-[(C₁-C₄)-alkyl]-amino und
R⁵, R⁶ unabhängig voneinander Wasserstoff oder (C₁-C₄)-Alkyl bedeuten,
oder deren physiologisch verträglichen Salze mit Säuren oder, falls mindestens einer der Reste R⁵ und R⁶ Wasserstoff ist, mit Basen,
dadurch gekennzeichnet, daß man Verbindungen der Formel II, 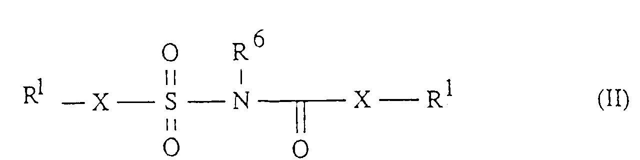 worin R¹, R⁶ und X wie in Formel I definiert sind, mit Verbindungen der Formel III, 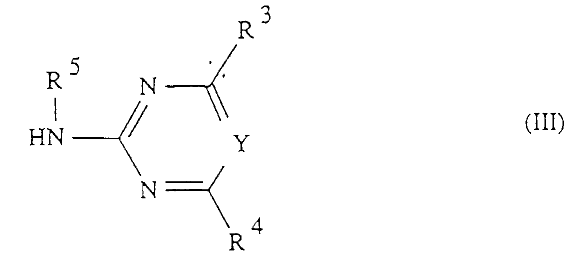 worin R³, R⁴, R⁵ und Y wie in Formel I definiert sind, umsetzt.

2. Verfahren nach Anspruch 1, dadurch gekennzeichnet, daß R¹X N-(C₁-C₆)-Alkylsulfonyl-N-(C₁-C₃)-alkyl-amino oder (C₁-C₄)-Alkoxyphenoxy, R³ und R⁴ unabhängig voneinander (C₁-C₂)-Alkyl oder (C₁-C₂)-Alkoxy und R⁵ und R⁶ Wasserstoff oder Methyl bedeuten.

3. Verfahren nach Anspruch 1 oder 2, dadurch gekennzeichnet, daß R¹X N-[(C₁-C₃)-Alkylsulfonyl]-N-[(C₁-C₂)-alkyl]-amino oder (C₁-C₃)-Alkoxyphenoxy und R³ und R⁴ unabhängig voneinander (C₁-C₂)-Alkyl oder (C₁-C₂)-Alkoxy und R⁵ und R⁶ Wasserstoff oder Methyl bedeuten.

4. Verfahren nach cinem oder mehreren der Ansprüche 1 bis 3, dadurch gekennzeichnet, daß man das Verfahren in Gegenwart eines unter den Reaktionsbedingungen inerten anorganischen oder organischen Lösungsmittels oder Gemischen mehrerer entsprechender Lösungsmittel durchführt.

5. Verfahren nach einem oder mehreren der Ansprüche 1 bis 4, dadurch gekennzeichnet, daß das Verfahren in Gegenwart eines inerten organischen Lösungsmittels durchgeführt wird.

6. Verfahren nach einem oder mehreren der Ansprüche 1 bis 5, dadurch gekennzeichnet, daß die Verbindung der Formel II irn Verhältnis zur Verbindung der Formel III äquimolar oder in geringem Überschuß eingesetzt wird.

7. Verfahren nach Anspruch 6, dadurch gekennzeichnet, daß das Molverhältnis II:III im Bereich von 1:1 bis 1,2:1 ist.

8. Verfahren nach einem oder mehreren der Ansprüche 1 bis 7, dadurch gekennzeichnet, daß die Reaktionstemperaturen im Bereich von 0°C bis zum Siedepunkt des eingesetzten Lösungsmittels sind.

9. Verbindungen der Formel II,
R¹-X-SO₂-NR⁶-CO-X-R¹ (II)
worin
X -ONR²- oder -SO₂-NR²-, wobei O bzw. SO₂ aus den beiden letztgenannten divalenten Gruppen direkt an R¹ gebunden sind,
R¹ (C₁-C₆)-Alkyl, (C₂-C₆)-Alkenyl oder (C₂-C₆)-Alkinyl, wobei jeder der drei letztgenannten Reste unsubstituiert oder ein- oder mehrfach durch Reste aus derGruppe Halogen, (C₁-C₄)-Alkoxy und (C₁-C₄)-Alkoxycarbonyl substituiert ist,
R² Wasserstoff, (C₁-C₆)-Alkyl, (C₂-C₆)-Alkenyl, (C₂-C₆)-Alkinyl oder (C₃-C₆)-Cycloalkyl,
R⁶ Wasserstoff oder C₁-C₄-Alkyl
bedeuten.

10. Verfahren zur Herstellung von Verbindungen der Formel II nach Anspruch 9, dadurch gekennzeichnet, daß man Verbindungen der Formel IV,
R¹-X-H (IV)
worin R¹ wie in Formel II definiert ist, mit der etwa halbmolaren Menge an Chlorsulfonylisocyanat umsetzt.

11. Verfahren nach Anspruch 10, dadurch gekennzeichnet, daß man die Verbindung der Formel IV als freie (neutrale) Verbindung einsetzt.

12. Verfahren nach Anspruch 11, dadurch gekennzeichnet, daß man die Umsetzung in Gegenwart einer organischen oder anorganischen Base durchführt.

13. Verfahren nach Anspruch 10, dadurch gekennzeichnet, daß man die Verbindung IV als Mischung der freien (neutralen) Verbindung der Formel IV und einem Alkalimetallsalz der Verbindung IV im Molverhältnis von etwa 1:1 einsetzt.

14. Verfahren nach einem oder mehreren der Ansprüche 10 bis 13, dadurch gekennzeichnet, daß man die Verbindung der Formel IV zu dem Chlorsulfonylisocyanat im molaren Verhältnis von 1,0:0,5 bis 2,0:0,5 eingesetzt.

15. Verfahren nach einem oder mehreren der Ansprüche 10 bis 14, dadurch gekennzeichnet, daß man die Umsetzung in Gegenwart eines inerten organischen Lösungsmittels möglichst ohne Wasser durchführt.

16. Verfahren zur Herstellung einer Verbindung der Formel I, wie sie in Anspruch 1 definiert ist, dadurch gekennzeichnet, daß man
a) eine Verbindung der Formel IV,
R¹ - X - H (IV)
wobei R¹ wie in Formel I definiert ist, mit der etwa halbmolaren Menge an Chlorsulfonylisocyanat zu einer Verbindung der Formel II, 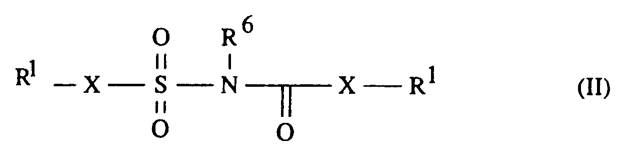 worin R¹, R⁶ und X wie in Formel I definiert sind,
umsetzt und
b) die erhaltene Verbindung der Formel II mit einer Verbindung der Formel III, 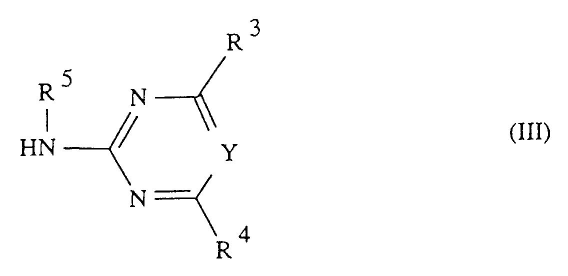 worin R³, R⁴, R⁵ und Y wie in Formel I definiert sind, anschließend umsetzt.

17. Verfahren nach Anspruch 16, dadurch gekennzeichent, daß das Verfahren ohne Zwischenisolierung der Verbindung der Formel II als Eintopfreaktion durchgeführt wird.

## Claims

1. A process for the preparation of a compound of the formula (I) or a salt thereof, 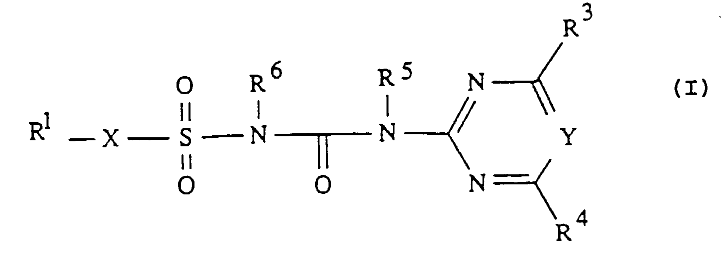 in which
X is oxygen, -O-NR²- or -SO₂-NR²-, the O or SO₂ of the two last-mentioned divalent groups being directly bound to R¹,
Y is nitrogen or CH,
R¹ is (C₁-C₆)-alkyl, (C₂-C₆)-alkenyl or (C₂-C₆)-alkynyl, each of said 3 radicals being unsubstituted or mono- or polysubstituted by radicals selected from the group consisting of halogen, (C₁-C₄)-alkoxy and (C₁-C₄)-alkoxycarbonyl,
or, where X = oxygen, is alternatively phenyl which is unsubstituted or substituted by one or more radicals selected from the group consisting of halogen, nitro, (C₁-C₄)-alkyl, (C₁-C₄)-haloalkyl, (C₁-C₄)-alkoxy, (C₁-C₄)-haloalkoxy or (C₁-C₄)-alkoxycarbonyl,
R² is hydrogen, (C₁-C₆)-alkyl, (C₂-C₆)-alkenyl, (C₂-C₆)-alkynyl or (C₃-C₆)-cycloalkyl,
R³, R⁴ are, independently of each other, hydrogen, (C₁-C₄)-alkyl or (C₁-C₄)-alkoxy, each of the last-mentioned two radicals being unsubstituted or mono- or polysubstituted by radicals selected from the group consisting of halogen, alkoxy and alkylthio, or halogen, (C₁-C₄)-alkylthio, (C₁-C₄)-alkylamino or di-[(C₁-C₄)-alkyl]amino and
R⁵, R⁶ are, independently of each other, hydrogen or (C₁-C₄)-alkyl,
or their physiologically tolerated salts with acids, or, where at least one of the radicals R⁵ and R⁶ is hydrogen, with bases,
which comprises reacting compounds of the formula II, 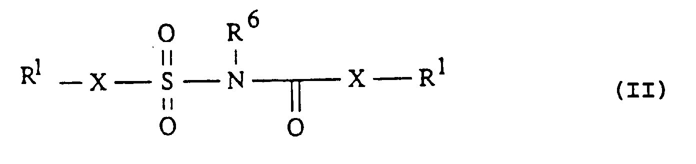 in which R¹, R⁶ and X are defined as in formula I, with compounds of the formula III, 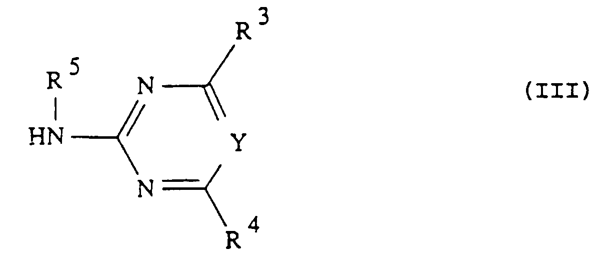 in which R³, R⁴, R⁵ and Y are defined as in formula I.

2. The process as claimed in claim 1, wherein R¹X is N-(C₁-C₆)-alkylsulfonyl-N-(C₁-C₃)-alkylamino or (C₁-C₄)-alkoxyphenoxy, R³ and R⁴ are, independently of each other, (C₁-C₂)-alkyl or (C₁-C₂) -alkoxy and R⁵ and R⁶ are each hydrogen or methyl.

3. The process as claimed in claim 1 or 2, wherein R¹X is N- [(C₁-C₃)-alkylsulfonyl] -N- [(C₁-C₂)-alkyl] amino or (C₁-C₃)-alkoxyphenoxy and R³ and R⁴ are, independently of each other, (C₁-C₂)-alkyl or (C₁-C₂)-alkoxy and R⁵ and R⁶ are each hydrogen or methyl.

4. The process as claimed in one or more of claims 1 to 3, wherein the process is carried out in the presence of an inorganic or organic solvent which is inert under the reaction conditions, or mixtures of a plurality of corresponding solvents.

5. The process as claimed in one or more of claims 1 to 4, wherein the process is carried out in the presence of an inert organic solvent.

6. The process as claimed in one or more of claims 1 to 5, wherein the compound of the formula II is used in an equimolar ratio to the compound of the formula III or in a slight excess.

7. The process as claimed in claim 6, wherein the molar ratio II:III is in the range from 1:1 to 1.2:1.

8. The process as claimed in one or more of claims 1 to 7, wherein the reaction temperatures are in the range from 0°C to the boiling point of the solvent used.

9. A compound of the formula II,
R¹-X-SO₂-NR⁶-CO-X-R¹ (II)
in which
X is -ONR²- or -SO₂-NR₂ -, the O or SO₂ of the two last-mentioned divalent groups being directly bound to R¹,
R¹ is (C₁-C₆)-alkyl, (C₂-C₆)-alkenyl or (C₂-C₆)-alkynyl, each of the three last-mentioned radicals being unsubstituted or mono- or polysubstituted by radicals selected from the group consisting of halogen, (C₁-C₄)-alkoxy and (C₁-C₄)-alkoxycarbonyl,
R² is hydrogen, (C₁-C₆)-alkyl, (C₂ -C₆)-alkenyl, (C₂-C₆)-alkynyl or (C₃-C₆)-cycloalkyl,
R⁶ is hydrogen or C₁-C₄-alkyl.

10. A process for the preparation of a compound of the formula II as claimed in claim 9, which comprises reacting a compound of the formula IV,
R¹-X-H (IV)
in which R¹ is defined as in formula II, with about half the molar amount of chlorosulfonyl isocyanate.

11. The process as claimed in claim 10, wherein the compound of the formula IV is used as the free (neutral) compound.

12. The process as claimed in claim 11, wherein the reaction is carried out in the presence of an organic or inorganic base.

13. The process as claimed in claim 10, wherein the compound IV is used as a mixture of the free (neutral) compound of the formula IV and an alkali metal salt of the compound IV in a molar ratio of about 1:1.

14. The process as claimed in one or more of claims 10 to 13, wherein the compound of the formula IV is used in a molar ratio to the chlorosulfonyl isocyanate of 1.0:0.5 to 2.0:0.5.

15. The process as claimed in one or more of claims 10 to 14, wherein the reaction is carried out in the presence of an inert organic solvent as far as possible without water.

16. A process for the preparation of a compound of the formula I, as defined in claim 1, which comprises
a) reacting a compound of the formula IV,
R¹-X-H (IV)
R¹ being defined as in formula I, with about half the molar amount of chlorosulfonyl isocyanate to give a compound of the formula II, 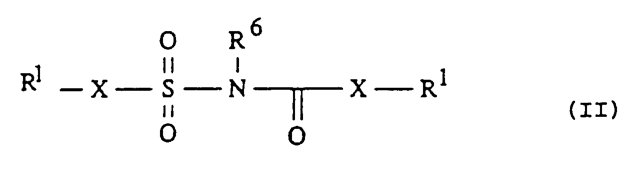 in which R¹, R⁶ and X are defined as in formula I, and
b) subsequently reacting the compound obtained of the formula II with a compound of the formula III, 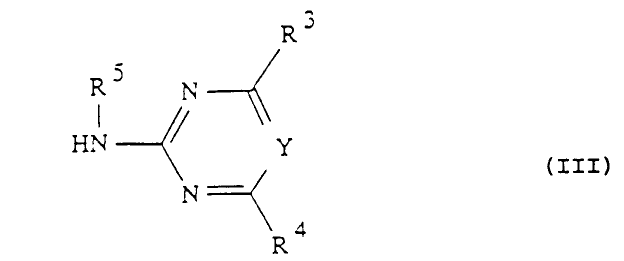 in which R³, R⁴, R⁵ and Y are defined as in formula I.

17. The process as claimed in claim 16, wherein the process is carried out as a one-pot reaction without intermediate isolation of the compound of the formula II.

## Revendications

1. Procédé de préparation de composés de formule (I) ou de leurs sels 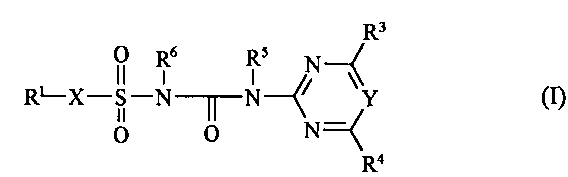 dans laquelle
X représente un atome d'oxygène ou un groupe -O-NR²- ou -SO₂-NR²-, où O et SO₂ dans les deux derniers groupes divalents cités sont directement liés à R¹,
Y représente un atome d'azote ou le groupe CH,
R¹ représente un reste alkyle en C₁-C₆, alcényle en C₂-C₆ ou alcynyle en C₂-C₆, chacun des 3 restes cités étant non substitué ou substitué une ou plusieurs fois par des restes du groupe constitué par les atomes d'halogène et les restes alcoxy en C₁-C₄ et (alcoxy en C₁-C₄)carbonyle, ou, dans le cas où X représente un atome d'oxygène, R¹ représente aussi un reste phényle non substitué ou substitué par un ou plusieurs restes du groupe constitué par les atomes d'halogène et les restes nitro, alkyle en C₁-C₄, halogénoalkyle en C₁-C₄, alcoxy en C₁-C₄, halogénoalcoxy en C₁-C₄, ou (alcoxy en C₁-C₄)carbonyle,
R² représente un atome d'hydrogène ou un reste alkyle en C₁-C₆, alcényle en C₂-C₆, alcynyle en C₂-C₆ ou cycloalkyle en C₃-C₆,
R³,R⁴ sont, indépendamment l'un de l'autre, un atome d'hydrogène, un reste alkyle en C₁-C₄ ou alcoxy en C₁-C₄, chacun des deux derniers restes cités étant non substitué ou substitué une ou plusieurs fois par des restes du groupe constitué par les atomes d'halogène et les restes alcoxy et alkylthio, ou un atome d'halogène ou un reste alkylthio en C₁-C₄, alkylamino en C₁-C₄ ou di(alkyl en C₁-C₄)amine, et
R⁵,R⁶ représentent indépendamment l'un de l'autre un atome d'hydrogène ou un reste alkyle en C₁-C₄,
ou de leurs sels physiologiquement acceptables avec des acides, ou, dans le cas où au moins l'un des restes R⁵ et R⁶ est l'hydrogène, avec des bases,
caractérisé en ce que l'on fait réagir des composés de formule II 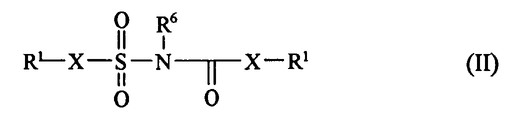 dans laquelle R¹, R⁶ et X ont la définition donnée pour la formule I, avec des composés de formule III 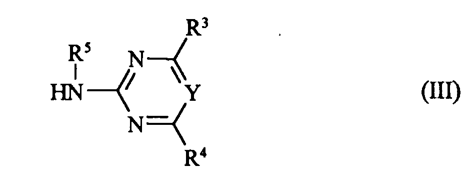 dans laquelle R³, R⁴, R⁵ et Y ont la définition donnée pour la formule I.

2. Procédé selon la revendication 1, caractérisé en ce que R¹X est un reste N-(alkylsulfonyl en C₁-C₆)-N-(alkyl en C₁-C₃)amine ou (alcoxy en C₁-C₄)phénoxy, R³ et R⁴ sont indépendamment l'un de l'autre un reste alkyle en C₁-C₂ ou alcoxy en C₁-C₂ et R⁵ et R⁶ représentent un atome d'hydrogène ou un reste méthyle.

3. Procédé selon la revendication 1 ou 2, caractérisé en ce que R¹X est un reste N-(alkylsulfonyl en C₁-C₃)-N-(alkyl en C₁-C₂)amino ou (alcoxy en C₁-C₃)phénoxy, R³ et R⁴ sont indépendamment l'un de l'autre un reste alkyle en C₁-C₂ ou alcoxy en C₁-C₂ et R⁵ et R⁶ représentent un atome d'hydrogène ou un reste méthyle.

4. Procédé selon l'une ou plusieurs des revendications 1 à 3, caractérisé en ce que l'on effectue le procédé en présence d'un solvant inorganique ou organique inerte dans les conditions de la réaction ou en présence d'un mélange de plusieurs solvants correspondants.

5. Procédé selon l'une ou plusieurs des revendications 1 à 4, caractérisé en ce que l'on effectue le procédé en présence d'un solvant organique inerte.

6. Procédé selon l'une ou plusieurs des revendications 1 à 5, caractérisé en ce que l'on utilise le composé de formule II en une quantité équimolaire ou en léger excès par rapport au composé de formule III.

7. Procédé selon la revendication 6, caractérisé en ce que le rapport molaire II:III est compris entre 1:1 et 1,2:1.

8. Procédé selon l'une ou plusieurs des revendications 1 à 7, caractérisé en ce que les températures réactionnelles sont comprises entre 0°C et le point d'ébullition du solvant utilisé.

9. Composés de formule II
R¹-X-SO₂-NR⁶-CO-X-R¹ (II)
dans laquelle
X représente un groupe -ONR²- ou -SO₂-NR²-, où O et SO₂ dans les deux derniers groupes divalents cités sont directement liés à R¹,
R¹ représente un reste alkyle en C₁-C₆, alcényle en C₂-C₆ ou alcynyle en C₂-C₆, chacun des 3 derniers restes cités étant non substitué ou substitué une ou plusieurs fois par des restes du groupe constitué par les atomes d'halogène et les restes alcoxy en C₁-C₄ et (alcoxy en C₁-C₄)carbonyle,
R² représente un atome d'hydrogène ou un reste alkyle en C₁-C₆, alcényle en C₂-C₆, alcynyle en C₂-C₆ ou cycloalkyle en C₃-C₆,
R⁶ représente un atome d'hydrogène ou un reste alkyle en C₁-C₄.

10. Procédé de préparation de composés de formule II selon la revendication 9, caractérisé en ce que l'on fait réagir des composés de formule IV
R¹-X-H (IV)
dans laquelle R¹ a la définition donnée pour la formule II, avec à peu près la moitié de la quantité en moles d'isocyanate de chlorosulfonyle.

11. Procédé selon la revendication 10, caractérisé en ce que l'on utilise le composé de formule IV sous forme de composé libre (neutre).

12. Procédé selon la revendication 11, caractérisé en ce que l'on effectue la réaction en présence d'une base organique ou inorganique.

13. Procédé selon la revendication 10, caractérisé en ce que l'on utilise le composé IV sous forme d'un mélange du composé de formule IV libre (neutre) et d'un sel de métal alcalin du composé IV en un rapport molaire d'environ 1:1.

14. Procédé selon l'une ou plusieurs des revendications 10 à 13, caractérisé en ce que l'on utilise un rapport molaire du composé de formule IV à l'isocyanate de chlorosulfonyle de 1,0:0,5 à 2,0:0,5.

15. Procédé selon l'une ou plusieurs des revendications 10 à 14, caractérisé en ce que l'on effectue la réaction en présence d'un solvant organique inerte, le plus possible sans eau.

16. Procédé de préparation d'un composé de formule I, tel qu'il est défini dans la revendication 1, caractérisé en ce que
a) on fait réagir un composé de formule IV
R¹-X-H (IV)
dans laquelle R¹ a la définition donnée pour la formule I, avec à peu près la moitié de la quantité en moles d'isocyanate de chlorosulfonyle, pour former un composé de formule II 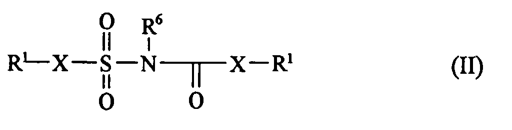 dans laquelle R¹, R⁶ et X ont la définition donnée pour la formule I, et
b) on fait ensuite réagir le composé de formule II obtenu avec un composé de formule III 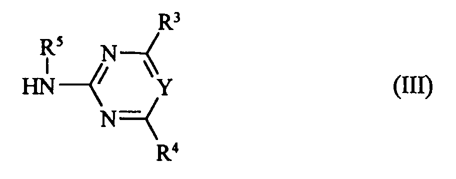 dans laquelle R³, R⁴, R⁵ et Y ont la définition donnée pour la formule I.

17. Procédé selon la revendication 16, caractérisé en ce que l'on effectue le procédé sous forme d'une réaction dans un seul récipient sans isolement intermédiaire du composé de formule II.
